# EUROPÄISCHE PATENTSCHRIFT

(11) **EP 2 146 678 B1**
(45) Veröffentlichungstag und Bekanntmachung des Hinweises auf die Patenterteilung: **06.07.2011**
(21) Anmeldenummer: 08717978.4
(22) Anmeldetag: 18.03.2008
(51) Int. Cl.: A61K 6/027

(54) **NIOBHALTIGES INFILTRATIONSGLAS**
INFILTRATION GLASS CONTAINING NIOBIUM
VERRE D'INFILTRATION CONTENANT DU NIOBIUM

(30) Priorität: 22.03.2007 EP 07104702
(43) Veröffentlichungstag der Anmeldung: 27.01.2010
(73) Patentinhaber: Vita Zahnfabrik H. Rauter GmbH & Co. KG, 79704 Bad Säckingen (DE)
(72) Erfinder: STEPHAN, Marc, 79539 Lörrach (DE); DURSCHANG, Bernhard, 97228 Rottendorf (DE)
(74) Vertreter: von Kreisler Selting Werner
(86) Internationale Anmeldenummer: PCT/EP2008/053247
(87) Internationale Veröffentlichungsnummer: WO 2008/113810

(56) Entgegenhaltungen:
- WO-A-2005/115936
- ANIL SAIGAL: "Modelling of residual stresses and mechanical behavior of glass-infiltrated spinel ceramic composites" COMPUTATIONAL MODELLING OF MATERIALS, MINERALS AND METALS PROCESSING, PROCEEDINGS OF CONFERENCE, 2001, Seiten 643-651, XP008082072 Warrendale, PA, USA

## Beschreibung

Die vorliegende Erfindung betrifft ein niobhaltiges Infiltrationsglas, insbesondere für keramische Körper, einen keramischen Körper einsetzbar als vollkeramischer Zahnersatz, bei dem ein poröses keramisches Gerüstmaterial mit einem Glas infiltriert wird.

Vollkeramische Massen werden heutzutage im Frontzahnbereich, als Inlays, Onlays, Veneers und Seitenzahnbrücken eingesetzt.

EP-B-0 241 384 beschreibt ein Verfahren, bei dem Metalloxidpartikel porös gesintert werden und das entstandene Gerüst anschließend mit einem Glas infiltriert wird. Das gesinterte Gerüst besteht hauptsächlich aus Aluminiumoxid und/oder Zirkonoxid. Als Infiltrationsglas wird ein alkalisches Borosilicatglas, das Aluminiumoxid enthält, beschrieben. In den Beispielen ist eine der Hauptkomponenten des Glases Lanthanoxid. Der Ausdehnungskoeffizient soll leicht unterhalb des porösen Unterbaus liegen.

Das DE-B-100 61 630 offenbart ein Verfahren, bei dem Cer-stabilisiertes Zirkonoxid und Mischungen von Cer-stabilisierten . Zirkonoxid mit Aluminiumoxid als Gerüstmaterial verwendet werden. Außerdem wird ein Glas mit der Zusammensetzung 15-35 Gew.% La₂O₃, 10-25 Gew.% SiO₂, 10-25 Gew.% AI₂O₃, 5-20 Gew.% B₂O₃, 5-20 Gew.% CaO, 0-10 Gew% ZrO₂, 0-10 Gew.% TiO₂ und 0-15 Gew.% CeO₂ beansprucht, sowie Zusätze an weiteren ZrO₂-stabilisierenden Metalloxiden, insbesondere 0-10 Gew.% MgO und 0-10 Gew.% Y₂O_{3.}

DE 692-T-06 256 beschreibt des weiteren Gerüstmaterialien auf Basis von Aluminium-/Magnesiumoxid-Spinellen.

Die WO-A-2005/115936 betrifft ein Glas- oder Glaskeramikpulver, umfassend Multikomponentengläser mit mindestens drei Elementen, welches dadurch gekennzeichnet ist, dass das Glas- oder Glaskeramikpulver eine mittlere Partikelgröße < 1 µm, vorzugsweise < 0,1 µm, besonders bevorzugt <10 nm, aufweist. Offenbart werden Glaszusammensetzungen aus 5 Gew. % SiO₂, 20 Gew. % B₂O₃, 2,5 Gew. % TiO₂, 2,5 Gew. % ZrO₂, 20 Gew. % ZnO, 35 Gew. % La₂O₃, 5 Gew. % WO₃ und 10 Gew. % Nb₂O₅, die für die Verwendung im Bereicht Infiltrationsgläser und Dentalkeramiken vorgesehen sind.

Anil Saigal et al. beschreiben in Computational Modelling of Materials, Minerals and Metals Processing, herausgegeben von M. Cross, J.W. Evans und C. Bailey, TMS (The Minerals, Metals & Materials Society), 2001, Infiltrationsgläser aus La₂O₃-Al₂O₃-B₂O₃-SiO₂, in denen kein Niob enthalten ist.

Der Erfindung liegt die Aufgabe zugrunde, ein Glas zu entwickeln, dass sich in die bekannten vollkeramische Gerüste infiltrieren lässt und verbesserte mechanische und chemische Beständigkeiten besitzt. Die bislang eingesetzten Gläser sind durch verschiedene Randbedingungen eingeschränkt. Die Infiltrationstemperatur muss unterhalb der Sintertemperatur des Gerüstes liegen, am besten unterhalb 1200 °C. Der Ausdehnungskoeffizient muss geringfügig unter dem Wert des Gerüstes liegen. Das Glas darf keine ungewünschte Farbe aufweisen. Das Glas muss sich fehlerfrei infiltrieren lassen. Das Glas soll hohe mechanische und chemische Beständigkeiten aufweisen. Insbesondere die Limitierung der Infiltrationstemperatur und damit der Viskosität der glasbildenden Schmelze stehen der chemischen und mechanischen Stabilität entgegen, da Gläser mit einem hohen Netzwerkbildneranteil (z. B. SiO₂, B₂O₃) sehr gute mechanische und chemische Beständigkeiten aufweisen, aber erst bei höheren Temperaturen die notwendige Viskosität von ca. 10³ dPas erreichen.

Das bislang verwendete hoch-lanthan-haltige Glas lässt sich zwar hervorragend infiltrieren, zeigt bei einer exakten Anpassung im Ausdehnungsverhalten gute mechanische Eigenschaften, ist im Bereich der chemischen Stabilität im Sinne der Dentalnormen jedoch nur als Gerüstmaterial ausreichend.

Überraschenderweise zeigte sich, dass die Verwendung von Nioboxid sowohl als Zusatz zu lanthan-haltigen Gläsern als auch als Bestandteil in weiteren Silicat-Systemen eine deutliche Verbesserung in der chemischen Beständigkeit hervorruft. Das der Erfindung zu Grunde liegende Problem wird gelöst durch ein Infiltrationsglas für keramische Körper auf silicatischer Basis, aus dem System SiO₂-La₂O₃-AbO₃-B₂O₃ mit 15-35 Gew.% La₂O₃, 10-25 Gew.% SiO₂, 10-25 Gew.% Al₂O₃, 5-20 Gew.% B₂O₃, 5-20 Gew.% CaO, 0-10 Gew.% ZrO₂, 0-10 Gew.% TiO₂ und 0-15 Gew.% CeO₂ zusätzlich Nb₂O₅ in einer Menge von 0,1-20 Gew.%.

Das erfindungsgemäße Infiltrationsglas zeigte bei Auslagerungen als definierter Glasgrieß (Kornfraktion 100-200 um, in Anlehnung an die DIN ISO 719) in 4 % Essigsäure bei 80 °C für 16 h eine Verringerung der Glaslöslichkeit auf unter 3% der bekannten Gläser. Die mechanischen Eigenschaften und die weiteren Spezifikationen werden vom Nioboxid nicht negativ beeinflusst. Das erfindungsgemäße Infiltrationsglas weist insbesondere eine Löslichkeit von < 1100 µg/cm², insbesondere < 900 µg/cm² bzw.< 700 µg/cm² nach DIN EN ISO 6872 auf.

Das erfindungsgemäße Infiltrationsglas kann weitere Metalloxide in einer Oxidationsstufe, die zur Stabilisierung von ZrO₂ geeignet ist, enthalten, wobei insbesondere 0-10 Gew.% MgO und/oder 0-10 Gew.% Y₂O₃ zugemischt werden können.

Das erfindungsgemäße Infiltrationsglas kann weiterhin farbgebende Oxide enthalten.

Das erfindungsgemäße Infiltrationsglas kann typischerweise als Pulver angefertigt und vertrieben werden.

Das Pulver kann dann z.B. in an sich bekannter Weise zur Infiltration poröser Keramikkörper verwendet werden. Gegenstand der Erfindung sind mithin auch keramische Körper, die mit dem erfindungsgemäßen Infltrationsglas infiltriert worden sind, z.B. Dentalrestaurationen.

Gegenstand der Erfindung ist weiterhin auch die Verwendung des erfindungsgemäβen Glases als Infiltrationsglas für vollkeramische Dentalmassen. Nb₂O₅ kann insbesondere in Mengen von 0,1 bis 20 Gew.%, oder 5 bis 15 Gew.% in dem erfindungsgemäßen Infiltrationsglas vorhanden sein.

### Beispiele:

### Beispiel 1:

Ein Glas aus dem SiO₂-Nb₂O₅-Na₂O-B₂O₃-System mit Zusätzen von Aluminium-, Zirkon- und Ceroxid zur Verwendung für Gerüste aus Aluminiumoxid und Cer-stabilisierten Zirkonoxid, so dass es zu keinen nennenswerten Auflösungs- bzw. Abscheidungsprozessen vom Gerüst ins Glas, respektive aus dem Glas kommt.

Das Glas mit der Zusammensetzung 29,5 Gew.% SiO₂, 28,7 Gew.% Nb₂O₅, 12,8 Gew.% Na₂O, 6,8 Gew.% B₂O₃, 8,0 Gew.% Al₂O₃, 6,6 Gew% ZrO₂ und 7,6 Gew.% CeO₂ zeigt nach einer Auslagerung in 4 % Essigsäure nach 16 h bei 80 °C einen Gewichtsverlust von 3,5 mg pro Gramm Einwaage. Der Ausdehnungskoeffizient α₂₀₋₃₀₀ liegt bei 8,3·10⁻⁶ K⁻¹, angepasst an ein Gerüst von 50 Gew.% Cer-stabilisiertes Zirkonoxid und 50 Gew.% Aluminiumoxid. Das momentan am häufigsten eingesetzte Infiltrationsglas für dentale Vollkeramiken (In Ceram^{©}-Zirconia-Glas) hat einen Gewichtsverlust von ca. 120 mg/g_{Einwaage}.

### Beispiel 2:

Das Glas mit der Zusammensetzung 29,9 Gew.% SiO₂, 37,0 Gew.% Nb₂O₅, 11,1 Gew.% Na₂O, 7,5 Gew.% K₂O, 6,7 Gew% ZrO₂ und 7,7 Gew.% CeO₂ zeigt nach einer Auslagerung in 4 % Essigsäure nach 16 h bei 80 °C einen Gewichtsverlust von 9,2 mg pro Gramm Einwaage. Der Ausdehnungskoeffizient a₂₀₋₃₀₀ liegt bei 9,3·10⁻⁶ K⁻¹ ist in diesem Fall an ein reines Cer-stabilisiertes-Zirkonoxid-Gerüst angepasst.

### Beispiel 3:

Das Glas mit der Zusammensetzung 21,5 Gew.% SiO₂, 11,0 Gew.% B₂O₃, 17,5 Gew.% AI₂O₃, 5,5 Gew.% CaO, 31 Gew.% La₂O₃, 4 Gew.% TiO₂, 9,5 Gew.% Nb₂O₅ zeigt eine chemische Löslichkeit von 653 µg/cm² nach DIN EN ISO 6872 nach Infiltration in einen VITA^{©} In-Ceram Classic ALUMINA BLANK^{©}. Im Vergleich hierzu zeigt das bisher auf dem Markt erhältliche VITA In-Ceram^{©} ALUMINA GLASS POWDER eine chemische Löslichkeit von etwa 1200 µg/cm²_{.}

## Patentansprüche

1. Infiltrationsglas, insbesondere für keramische Körper auf silicatischer Basis, , aus dem System SiO₂-La₂O₃-Al₂O₃-B₂O₃ mit 15-35 Gew.% La₂O₃, 10-25 Gew.% SiO₂, 10-25 Gew.% Al₂O₃, 5-20 Gew.% B₂O₃, 5-20 Gew.% CaO, 0-10 Gew.% ZrO₂, 0-10 Gew.% TiO₂ und 0-15 Gew.% CeO₂ mit einer Zugabe von Nb₂O₅ von 0,1-20 Gew.%.

2. Infiltrationsglas nach Anspruch 1, wobei das Infiltrationsglas weitere Metalloxide in einer Oxidationsstufe zur Stabilisierung von ZrO₂ enthält.

3. Infiltrationsglas nach mindestens einem der Ansprüche 1 oder 2, wobei das Infiltrationsglas 0-10 Gew.% MgO und/oder 0-10 Gew.% Y₂O₃ enthält.

4. Infiltrationsglas nach mindestens einem der Ansprüche 1 bis 3, wobei das Infiltrationsglas farbgebende Oxide enthält.

5. Infiltrationsglas nach mindestens einem der Ansprüche 1 bis 4 mit einer Löslichkeit von < 1100 µg/cm² nach DIN EN ISO 6872.

6. Keramischer Körper erhältlich durch Infiltration eines porösen keramischen Körpers mit einem Infiltrationsglas nach mindestens einem der Ansprüche 1 bis 5.

7. Keramischer Körper nach Anspruch 6, **dadurch gekennzeichnet, dass** der keramische Körper eine Dentalrestauration ist.

8. Verwendung von niobhaltigem Infiltrationsglas nach mindestens einem der Ansprüche 1 bis 5 als Infiltrationsglas für keramische Körper.

9. Verwendung von niobhaltigem Infiltrationsglas nach mindestens einem der Ansprüche 1 bis 5 als Infiltrationsglas für vollkeramische Dentalmassen.

## Claims

1. An infiltration glass, especially for silicate-based ceramic bodies, from the system SiO₂-La₂O₃-Al₂O₃-B₂O₃ with 15-35% by weight La₂O₃, 10-25% by weight SiO₂, 10-25% by weight Al₂O₃, 5-20% by weight B₂O₃, 5-20% by weight CaO, 0-10% by weight ZrO₂, 0-10% by weight TiO₂ and 0-15% by weight CeO₂ with an addition of Nb₂O₅ of from 0.1 to 20% by weight.

2. The infiltration glass according to claim 1, wherein said infiltration glass contains further metal oxides at an oxidation stage suitable for the stabilization of ZrO₂.

3. The infiltration glass according to at least one of claims 1 or 2, wherein said infiltration glass contains from 0 to 10% by weight MgO and/or from 0 to 10% by weight Y₂O₃.

4. The infiltration glass according to at least one of claims 1 to 3, wherein said infiltration glass contains coloring oxides.

5. The infiltration glass according to at least one of claims 1 to 4 having a solubility of < 1100 µg/cm² according to DIN EN ISO 6872.

6. A ceramic body obtainable by infiltration of a porous ceramic body with an infiltration glass according to at least one of claims 1 to 5.

7. The ceramic body according to claim 6, **characterized in that** said ceramic body is a dental restoration.

8. Use of niobium-containing infiltration glass according to at least one of claims 1 to 5 as an infiltration glass for ceramic bodies.

9. Use of niobium-containing infiltration glass according to at least one of claims 1 to 5 as an infiltration glass for all-ceramic dental compositions.

## Revendications

1. Verre d'infiltration, en particulier pour des corps céramiques à base de silicate, issu du système SiO₂-La₂O₃-Al₂O₃-B₂O₃ avec 15 à 35 % en poids de La₂O₃, 10 à 25 % en poids de SiO₂, 10 à 25 % en poids de Al₂O₃, 5 à 20 % en poids de B₂O₃, 5 à 20 % en poids de CaO, 0 à 10 % en poids deZrO₂, 0 à 10 % en poids de TiO₂ et 0 à 15 % en poids de CeO₂ avec un apport de Nb₂O₅ de 0,1 à 20 % en poids.

2. Verre d'infiltration selon la revendication 1, le verre d'infiltration contenant d'autres oxydes métalliques dans une étape d'oxydation pour la stabilisation de ZrO₂.

3. Verre d'infiltration selon au moins l'une des revendications 1 ou 2, le verre d'infiltration contenant 0 à 10 % en poids de MgO et/ou 0 à 10 % en poids de Y₂O₃.

4. Verre d'infiltration selon au moins l'une des revendications 1 à 3, le verre d'infiltration contenant des oxydes colorants.

5. Verre d'infiltration selon au moins l'une des revendications 1 à 4, avec une solubilité de < 1100 µg/cm² selon la norme DIN EN ISO 6872.

6. Corps céramique pouvant être obtenu par infiltration d'un corps céramique poreux avec un verre d'infiltration selon au moins l'une des revendications 1 à 5.

7. Corps céramique selon la revendication 6, **caractérisé en ce que** le corps céramique est une restauration dentaire.

8. Utilisation de verre d'infiltration contenant du niobium selon au moins l'une des revendications 1 à 5 en tant que verre d'infiltration pour corps céramiques.

9. Utilisation de verre d'infiltration contenant du niobium selon au moins l'une des revendications 1 à 5 en tant que verre d'infiltration pour masses dentaires entièrement en céramique.
